(19) Europäisches Patentamt
European Patent Office
Office européen des brevets

(11) EP 0 887 077 A1

(12) **EUROPÄISCHE PATENTANMELDUNG**

(43) Veröffentlichungstag:
**30.12.1998 Patentblatt 1998/53**

(51) Int. Cl.[6]: **A61K 31/015**

(21) Anmeldenummer: **97110534.1**

(22) Anmeldetag: **27.06.1997**

(84) Benannte Vertragsstaaten:
**AT BE CH DE DK ES FI FR GB GR IE IT LI LU MC NL PT SE**

(71) Anmelder:
**BOEHRINGER MANNHEIM GMBH**
**68298 Mannheim (DE)**

(72) Erfinder:
- **Dickhaut, Joachim Dr., Dipl.-Chemiker**
  **69121 Heidelberg (DE)**
- **Haag, Rainer Dr., Dipl.-Chemiker**
  **68526 Ladenburg (DE)**
- **Grams, Frank Dr., Dipl.-Chemiker**
  **68219 Mannheim (DE)**

(54) **Verwendung von Azulenderivaten als Metalloproteaseinhibitoren**

(57) Gegenstand der vorliegenden Erfindung ist die Verwendung von Azulenderivaten sowie deren Tautomere, Enantiomere, Diastereomere, Racemate und physiologisch verträglichen Salze oder Ester als Metalloproteaseinhibitoren.

EP 0 887 077 A1

## Beschreibung

Die vorliegende Erfindung betrifft die Verwendung von Azulenderivaten sowie deren Tautomere, Enantiomere, Diastereomere, Racemate und physiologisch verträglichen Salze oder Ester als Metalloproteaseinhibitoren.

Azulene sind unter anderem als Inhaltsstoffe der Kamille seit langem bekannt. Überraschender-weise wurde gefunden, daß Azulenderivate als Metalloproteaseinhibitoren wirken.
Metalloproteasen spielen in vielen physiologischen und pathophysiologischen Prozessen eine große Rolle.
Beispiele dafür sind das Angiotensin Converting Enzyme (ACE) und die neutrale Endopeptidase (NEP, EC 3.4.24.11), die am Metabolismus einer Reihe von blutdruckregulierenden Peptiden (z. B. Angiotensin I und ANF (atrial natriuretic factor)) beteiligt sind. ACE katalysiert die Spaltung des Angiotensin I zu dem blutdrucksteigernden Angiotensin II. NEP ist für den Abbau des vasodilatierenden Peptids ANF veranwortlich. Das Endothelin Converting Enzyme (ECE) spaltet das endogene, inaktive big-Endothelin zu dem effektiven Vasokonstriktor Endothelin-1, einem aus 21 Aminosäuren bestehenden Peptid. Die Inhibierung dieser Enzyme hat eine große therapeutische Bedeutung zur Behandlung des Bluthochdrucks, der Herzinsuffzienz, des Nierenversagens und des Schlaganfalls. BMP-1 (bone morphogenic factor 1) wurde als Metalloprotease erkannt, die bei der Umwandlung von Procollagen in fibrilläres Collagen eine Rolle spielt. Inhibitoren dieses Enzyms sind für die Behandlung von Fibrosen und sklerotischen Prozessen geeignet und können auch die Narbenbildung bei der Wundheilung günstig beeinflussen. (*Proc. Natl. Acad. Sci. USA* **1996**, 93, 5127, *Science* **1996**, *271*, 360).

Während Inhibitoren des ACE bereits therapeutisch angewandt werden (z. B. Captopril, Enalapril, (*Exp. Opin. Ther. Pat.* **1996**, *6*, 1147, sind für die Metalloproteasen wie NEP, ECE bisher keine klinisch verwendbaren Wirkstoffe bekannt, die frei von unerwünschten Nebenwirkungen und oral verfügbar sind. (Literaturübersichten: NEP: *Pharmacol. Rev* **1993**, *45*, 87; ECE: *Bioorg. Med Chem. Lett.* **1996**, *6*, 2317, zit. Lit. zu Inhibitoren vom Phosporamidontyp. Für das BMP-1 sind bisher noch keine niedermolekularen Inhibitoren bekannt.

Eine weitere Gruppe der Metalloproteasen ist die der Matrixmetalloproteasen (MMPs). In normalem Gewebe besteht ein Gleichgewicht zwischen Auf- und Abbau der extrazellulären Matrix. Die extrazelluläre Matrix wird von wenigstens drei Gruppen Proteasen, nämlich den Collagenasen, Gelatinasen und den Stromelysinen abgebaut. Normalerweise sorgen spezifische Inhibitoren dieser Enzyme, wie z.B. $\alpha_2$-Makroglobulin und TIMP (tissue inhibitor of metalloproteases) dafür, daß kein übermäßiger Abbau der extrazellulären Matrix stattfindet. Eine verwandte Gruppe von Proteasen ist die der Adamalysine. Ein Mitglied dieser Gruppe ist das TNF-$\alpha$ converting enzyme (TACE) (Moss et al., *Nature* **1996**, *385*, 733).
Wenigstens 11 verschiedene und doch sehr homologe Matrixmetalloproteinasen wurden charakterisiert, darunter die interstitial fibroblast collagenase (MMP-1, HFC), die neutrophile Collagenase (MMP-8, HNC), zwei Gelatinasen, Stromelysine (z.B. HSL-1) und Matrilysin (Birkedal-Hansen, H., Moore, W.G.I., Bodden, M.K. Windsor, L.J., Birkedahl-Hansen,B., DeCarlo, A., Engler, J.A., *Critical Rev. Oral Biol. Med.* **1993**, *4*, 197-250). Diese Proteinasen teilen eine Reihe struktureller und funktionaler Eigenschaften, unterscheiden sich allerdings in ihrer Substratspezifität. Nur HNC und HFC spalten natives triple-helikales Collagen der Typen I, II und III. Dabei entstehen Fragmente von 3/4 und 1/4 der ursprünglichen Länge. Durch diesen Abbau wird der Schmelzpunkt des Collagens erniedrigt. Anschließend kann es durch andere matrixabbauende Enzyme angegriffen werden.
Der unkontrollierte exzessive Abbau dieser Matrizes ist für viele pathologische Erscheinungsbilder, wie z.B. rheumatoide Arthritis, Osteoarthritis, Multiple Sklerose, Tumor Metastasierung, Cornea Ulzerationen, inflammatorische Prozesse und verschiedene Erkrankungen der Knochen und Zähne, typisch.
Die Pathogenese dieser Krankheiten sollte durch die Gabe von Metalloproteinaseinhibitoren positiv beeinflußt werden. In der Literatur finden sich inzwischen einige solche Verbindungen (eine Übersicht findet sich z.B. bei Nigel R. A. Beeley et al., *Curr. Opin. Ther. Pat.* **1994**, *4* (1), 7). Dabei handelt es sich vor allem um Peptide mit einem Hydroxamsäure-, Thiol- oder Phosphinrest als zinkbindende Gruppe (unter anderem z.B. WO-A-9209563 von Glycomed, EP-A-497192 von Hoffmann-LaRoche, WO-A-489577 von Celltech, EP-A-320118 von Beecham, US-A-4595700 von Searle).
Tumor Nekrose Faktor $\alpha$ (TNF$\alpha$) ist ein proinflammatorisches Zytokin, das bei einer Vielzahl von Erkrankungen pathogenetische Bedeutung besitzt. Klinisch wurde in einer multizentrischen, randomisierten Doppel-Blind-Studie von Elliot et al. in *Lancet* **1994**, *344*, 1105-1110 gezeigt, daß ein neutralisierender Antikörper gegen TNF$\alpha$ eine schnelle und ausgeprägte Besserung der Krankheitssymptome bei Patienten mit rheumatoiden Arthritis bewirken. Mittlerweile wurden von van Dullemen et al. in *Gastroenterology* **1995**, *109*, 129-135 klinische Daten publiziert, die eine therapeutische Wirkung solcher Antikörper bei Patienten mit Morbus Crohn belegen. Weiterhin wurde tierexperimentell gezeigt, daß Rolipram, das ebenfalls die Synthese von TNF$\alpha$ blockiert, in Tiermodellen für die Multiple Sklerose sehr gut wirksam ist. Thalidomid, eine weitere TNF$\alpha$-hemmende Substanz, wird klinisch zur Behandlung von chronischer Graft versus Host-Erkrankung, bei der Behandlung von Lepra nodulare, und von Patienten mit Lupus erythematodes eingesetzt. Zudem wurde für diese Substanz gezeigt, daß sie die Proliferation von HIV unterdrückt.

Bei folgenden Krankheitsbildern scheint TNF$\alpha$ von direkter pathogenetischer Bedeutung zu sein.
Degenerative Gelenkserkrankungen, rheumatoide Arthritis, Entzündung, Allergie, ARDS, Asthma, Herzinfarkt, Chronische Herzinsuffizienz, HIV-Infektion, Morbus Crohn, Ulzerative Colitis, Psoriasis, Dermatitis, Actinokeratose, Vaskulitiden, septischer Schock, Transplantatabstoßung, Multiple Sklerose, Ulzera, Diabetes, chronische Graft versus Host-Erkrankung, Lepra und andere Infektionskrankheiten Lupus Erythematodes, Paradontose und bei anderen Erkrankungen.

Der klinische Einsatz von monoklonalen Anti- TNFα-Antikörpern kann nur parenteral erfolgen. Das Arzneimittel ist teuer herzustellen und erfordert eine aufwendige Vertriebslogistik (Kühlkette, Lagerung, Verfallzeit, etc.). Zudem wird bei 50 % der Patienten, die zwischen 2 und 4 Injektionen bekommen haben, das Auftreten neutralisierender HACAs (Human anti-chimeric antibodies) festgestellt. Dies führt dazu, daß die beschwerdefreien Phasen immer kürzer werden. Die Entwicklung von Rolipram als Anti-TNFα-Therapieprinzip ist durch dessen emetische Wirkung beeinträchtigt. Die teratogenen Nebenwirkungen von Thalidomid und die schwache TNFα-Blockade lassen eine klinische Entwicklung dieser Substanz ebenfalls schwierig erscheinen.

Die Verbindungen, die Gegenstand der Erfindung sind, sind im folgenden näher beschrieben. Die Erfindung betrifft die Verwendung von Azulenderivaten insbesondere von Verbindungen der allgemeinen Formeln I und II als Metalloproteaseinhibitoren,

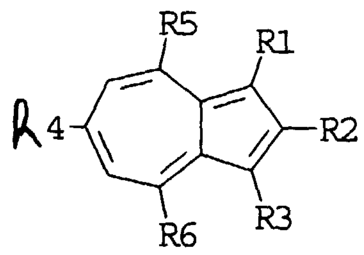

I

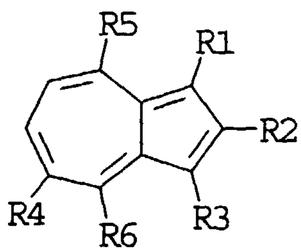

II

wobei

$R_1$, $R_2$ und $R_3$ einzeln oder unabhängig voneinander
Wasserstoff Hydroxy-, Amino-, Mercapto-, ein gerader oder verzweigter, gesättigter oder ungesättigter aliphatischer Rest mit 1-15 Kohlenstoffatomen, der ein oder mehrfach substituiert sein kann, eine Alkoxy-, Alkylamino-, Di-Alkylamino-, Alkylmercapto-, Alkylsulfinyl-, Alkylsufonyl-, Alkenyl-, Alkinyl-, Alkenyloxy-, Alkenylmercapto-, Alkinyloxy-, Alkinylmercapto-, Alkylcarbonylamino-, Di-N-Alkylcarbonylamino-, Alkylaminocarbonyl-, Formyl-, Alkylcarbonyl-, Carboxyl-, Alkoxycarbonyl-, Alkenyloxycarbonyl-, Alkinyloxycarbonyl-, Carboxyalkyl-, Alkyloxycarbonylalkyl, Alkenyloxycarbonylalkyl-, Alkinyloxycarbonylalkyl-, Nitro-, Cyano-, Halogen-, Trifluormethyl-, Azidogruppe, Phenyl, oder Phenylcarbonyl, das gegebenenfalls ein oder mehrfach unabhängig voneinander substituiert sein kann, einen carbocyclischen mono-, bi- oder tricyclischen Rest mit 7-15 C-Atomen oder ein heterocyclisches mono-, bi- oder tricyclisches Ringsystem bedeuten,

$R_4$ im Fall von Verbindungen der allgemeinen Formel I
Wasserstoff Halogen, $(CH_2)_nCH_2R_7$, $(CH_2)_nCHR_7R_8$, $CH(OR_8)R_7$, $CH(SR_8)R_7$, $NR_9R_{10}$, $OR_{10}$, $SR_{10}$, $C(O)R_{10}$,
im Fall von Verbindungen der allgemeinen Formel II
$R_7$, $(CH_2)_nCH_2R_7$, $(CH_2)_nCHR_7R_8$, $CH(OR_8)R_7$, $CH(SR_8)R_7$, $NR_9R_{10}$, $OR_{10}$, $SR_{10}$, $C(O)R_{10}$,

n die Zahl 0 oder 1 bedeutet

$R_5$ und $R_6$ einzeln und unabhängig voneinander
Wasserstoff, ein gerader oder verzweigter, gesättigter oder ungesättigter aliphatischer Rest mit 1-15 Kohlenstoffatomen, der ein oder mehrfach substituiert sein kann, eine Alkoxy-, Alkylamino, Di- Alkylamino-, Alkylmercapto-, Alkylsulfinyl-, Alkylsufonyl-, Alkenyl-, Alkinyl-, Alkenyloxy-, Alkenylmercapto-, Alkinyloxy-, Alkinylmercapto-, Alkylcarbonylamino-, Di- -Alkylcarbonylamino-, Alkylaminocarbonyl-, Formyl-, Alkylcarbonyl-, Carboxyl-, Alkoxycarbonyl-, Alkenyloxycarbonyl-, Alkinyloxycarbonyl-, Carboxyalkyl-, Alkyloxycarbonylalkyl, Alkenyloxycarbonylalkyl-, Alkinyloxycarbonylalkyl-, Nitro-, Cyano-, Halogen-, Trifluormethyl-, Azidogruppe, Phenyl, oder Phenylcarbonyl, das gegebenenfalls ein oder mehrfach unabhängig voneinander substituiert sein kann, einen carbocyclischen mono-, bi- oder tricyclischen Rest mit 7-15 C-Atomen oder ein heterocyclisches mono-, bi- oder tricyclisches Ringsystem,

$R_7$,
Alkyl-, Carboxyl-, Carboxoureido-, Alkoxycarbonyl-, Alkenyloxycarbonyl-, Alkinyloxycarbonyl-, Aminocarbonyl-, Alkylaminocarbonyl-, Alkenyloxycarbonylalkyl-, Alkinyloxycarbonylalkyl-, *N*-Hydroxyaminocarbonyl-, *N*-Alkoxyaminocarbonyl-, *N*-Alkyl-*N*-Hydroxyaminocarbonyl-, Hydroxamin-, *N*-Alkyl-*N*-Alkoxyaminocarbonyl-, Mercapto-, Alkylmercapto-, Phenylmercapto, Pyridylmercapto, Halogen-, Dihydroxyboryl-, Di-Alkoxyboryl-, Sulfonsäure-, Phosphonsäure-, Barbitursäure-,

$R_8$
Wasserstoff Alkyl-, Alkenyl-, Alkinyl-, Phenyl-, Pyridyl-, Thiophenyl-, Alkoxycarbonyl-, und

$R_9$
Wasserstoff Benzyl, Phenyl, Alkyl bedeutet,
und

$R_{10}$
Wasserstoff Carboxyl-, Alkoxycarbonyl-, Alkenyloxycarbonyl-, Alkinyloxycarbonyl-, Aminocarbonyl-, Alkylaminocarbonyl-, Alkenyloxycarbonylalkyl-, Alkinyloxycarbonylalkyl-, *N*-Hydroxyaminocarbonyl, *N*-Alkoxyaminocarbonyl-, *N*-Alkyl-*N*-Hydroxyaminocarbonyl-, *N*-Alkyl-*N*-Alkoxyaminocarbonyl- bedeutet

sowie deren Tautomere, Enantiomere, Diastereomere, Racemate und physiologisch verträglichen Salze oder Ester und Substanzen, die *in vivo* zu Verbindungen der Formel I bzw. II hydrolysiert oder metabolisiert werden.
Die Erfindung betrifft auch Verfahren zur Herstellung der obigen Verbindungen, Arzneimittel, die solche Verbindungen enthalten sowie die Verwendung dieser Verbindungen bei der Herstellung von Arzneimitteln.
In Verbindungen der allgemeinen Formel I und II muß mindestens einer der Reste $R_1$, $R_3$, $R_5$ oder $R_6$ ungleich Wasserstoff sein.
Bevorzugt sind Verbindungen der Formel I bzw. II worin

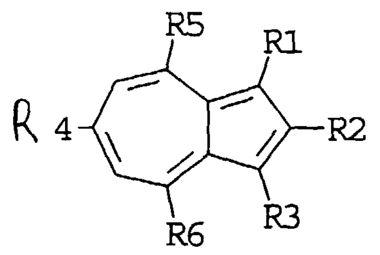

I

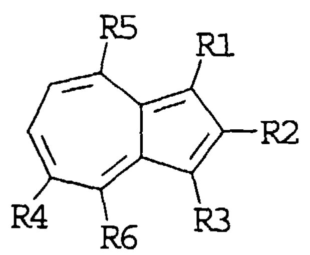

II

wobei

$R_1$, $R_2$ und $R_3$ einzeln oder unabhängig voneinander
Wasserstoff, Hydroxy-, Amino-, Mercapto-, Thio-, ein gerader oder verzweigter, gesättigter oder ungesättigter aliphatischer Rest mit 1-15 Kohlenstoffatomen, der ein oder mehrfach Hydroxy-, Amino-, Mercapto-, Thio-, $C_1$-$C_6$-Alkoxy-, $C_1$-$C_6$-Alkylamino-, Di-$C_1$-$C_6$-Alkylamino-, $C_1$-$C_6$-Alkylmercapto-, $C_1$-$C_6$-Alkylsulfinyl-, $C_1$-$C_6$-Alkylsulfonyl-, $C_2$-$C_6$-Alkenyl-, $C_2$-$C_6$-Alkinyl-, $C_2$-$C_6$-Alkenyloxy-, $C_2$-$C_6$-Alkenylmercapto-, $C_2$-$C_6$-Alkinyloxy-, $C_2$-$C_6$-Alkinylmercapto-, $C_1$-$C_6$-Alkylcarbonylamino-, $C_1$-$C_6$-Alkylaminocarbonyl-, Formyl-, $C_1$-$C_6$-Alkylcarbonyl-Carboxyl-, $C_1$-$C_6$-Alkoxycarbonyl-, $C_1$-$C_6$-Alkenyloxycarbonyl-, $C_1$-$C_6$-Alkinyloxycarbonyl-, Carboxy- $C_1$-$C_6$-alkyl-, $C_1$-$C_6$-Alkyloxycarbonyl-$C_1$-$C_6$-alkyl-, $C_2$-$C_6$-Alkenyloxycarbonyl- $C_1$-$C_6$-alkyl-, $C_2$-$C_6$-Alkinyloxycarbonyl- $C_1$-$C_6$-alkyl-, Benzyloxy-, Phenylmercapto-, Phenyloxy-, Nitro-, Cyano-, Halogen-, Trifluormethyl-, Azido-, Formylamino-, oder Phenylsubstituiert sein kann,
eine $C_1$-$C_6$-Alkoxy-, $C_1$-$C_6$-Alkylamino-, Di-$C_1$-$C_6$-Alkylamino-, $C_1$-$C_6$-Alkylmercapto-, $C_1$-$C_6$-Alkylsulfinyl, $C_1$-$C_6$-Alkylsufonyl-, $C_2$-$C_6$-Alkenyl-, $C_2$-$C_6$-Alkinyl-, $C_2$-$C_6$-Alkenyloxy-, $C_2$-$C_6$-Alkenylmercapto-, $C_2$-$C_6$-Alkinyloxy-, $C_2$-$C_6$-Alkinylmercapto-, $C_1$-$C_6$-Alkylcarbonylamino-, Di-$C_1$-$C_6$-Alkylcarbonylamino-, $C_1$-$C_6$-Alkylaminocarbonyl-, Formyl-, $C_1$-$C_6$-Alkylcarbonyl-, Carboxyl-, $C_1$-$C_6$-Alkoxycarbonyl-, $C_1$-$C_6$-Alkenyloxycarbonyl-, $C_1$-$C_6$-Alkinyloxycarbo-

nyl-, Carboxy- $C_1$-$C_6$-alkyl-, $C_1$-$C_6$-Alkyloxycarbonyl-$C_1$-$C_6$-alkyl-, $C_2$-$C_6$-Alkenyloxycarbonyl- $C_1$-$C_6$-alkyl-, $C_2$-$C_6$-Alkinyloxycarbonyl- $C_1$-$C_6$-alkyl-, Nitro-, Cyano-, Halogen-, Trifluormethyl-, Azidogruppe,
Phenyl, oder Phenylcarbonyl, das gegebenenfalls ein oder mehrfach unabhängig voneinander durch Hydroxy-, Amino-, Mercapto-, $C_1$-$C_6$-Alkoxy-, $C_1$-$C_6$-Alkylamino-, Di-$C_1$-$C_6$-Alkylamino-, $C_1$-$C_6$-Alkylmercapto-, $C_1$-$C_6$-Alkylsulfinyl-, $C_1$-$C_6$-Alkylsufonyl-, $C_2$-$C_6$-Alkenyl-, $C_2$-$C_6$-Alkinyl-, $C_2$-$C_6$-Alkenyloxy-, $C_2$-$C_6$-Alkenylmercapto-, $C_2$-$C_6$-Alkinyloxy-, $C_2$-$C_6$-Alkinylmercapto-, $C_1$-$C_6$-Alkylcarbonylamino-, $C_1$-$C_6$-Alkylaminocarbonyl-, Formyl-, $C_1$-$C_6$-Alkylcarbonyl-, Carboxyl-, $C_1$-$C_6$-Alkoxycarbonyl-, $C_1$-$C_6$-Alkenyloxycarbonyl-, $C_1$-$C_6$-Alkinyloxycarbonyl-, Carboxy- $C_1$-$C_6$-alkyl-, $C_1$-$C_6$-Alkyloxycarbonyl-$C_1$-$C_6$-alkyl-, $C_2$-$C_6$-Alkenyloxycarbonyl- $C_1$-$C_6$-alkyl-, $C_2$-$C_6$-Alkinyloxycarbonyl- $C_1$-$C_6$-alkyl-, Benzyloxy-, Phenylmercapto-, Phenyloxy-, Nitro-, Cyano-, Halogen-, Trifluormethyl-, Azido-, Formylamino-, Carboxy- oder Phenyl- substituiert sein kann,
einen carbocyclischen mono-, bi- oder tricyclischen Rest mit 7-15 C-Atomen oder ein heterocyclisches mono-, bi- oder tricyclisches Ringsystem darstellen kann, wobei die ungesättigten bzw. aromatischen Carbo- und Heterocyclen partiell oder vollständig hydriert sein können.

$R_4$ bedeutet im Fall von Verbindungen der allgemeinen Formel I
Wasserstoff Halogen, $(CH_2)_nCH_2R_7$, $(CH_2)_nCHR_7R_8$, $CH(OR_8)R_7$, $CH(SR_8)R_7$, $NR_9R_{10}$, $OR_{10}$, $SR_{10}$ oder $C(O)R_{10}$,
im Fall von Verbindungen der allgemeinen Formel II
$R_7$, $(CH_2)_nCH_2R_7$, $(CH_2)_nCHR_7R_8$, $CH(OR_8)R_7$, $CH(SR_8)R_7$, $NR_9R_{10}$, $OR_{10}$, $SR_{10}$ oder $C(O)R_{10}$.

n die Zahl 0 oder 1 bedeutet

$R_5$ und $R_6$ bedeuten einzeln und unabhängig voneinander
Wasserstoff einen geraden oder verzweigten, gesättigten oder ungesättigten aliphatischen Rest mit 1-15 Kohlenstoffatomen; der ein oder mehrfach Hydroxy-, Amino-, Mercapto-, $C_1$-$C_6$-Alkoxy-, $C_1$-$C_6$-Alkylamino-, Di-$C_1$-$C_6$-Alkylamino-, $C_1$-$C_6$-Alkylmercapto-, $C_1$-$C_6$-Alkylsulfinyl-, $C_1$-$C_6$-Alkylsulfonyl-, $C_2$-$C_6$-Alkenyl-, $C_2$-$C_6$-Alkinyl-, $C_2$-$C_6$-Alkenyloxy-, $C_2$-$C_6$-Alkenylmercapto-, $C_2$-$C_6$-Alkinyloxy-, $C_2$-$C_6$-Alkinylmercapto-, $C_1$-$C_6$-Alkylcarbonylamino-, $C_1$-$C_6$-Alkylaminocarbonyl-, Formyl-, $C_1$-$C_6$-Alkylcarbonyl-, Carboxyl-, $C_1$-$C_6$-Alkoxycarbonyl-, $C_1$-$C_6$-Alkenyloxycarbonyl-, $C_1$-$C_6$-Alkinyloxycarbonyl-, Carboxy- $C_1$-$C_6$-alkyl-, $C_1$-$C_6$-Alkyloxycarbonyl-$C_1$-$C_6$-alkyl-, $C_2$-$C_6$-Alkenyloxycarbonyl- $C_1$-$C_6$-alkyl-, $C_2$-$C_6$-Alkinyloxycarbonyl- $C_1$-$C_6$-alkyl-, Benzyloxy-, Phenylmercapto-, Phenyloxy-, Nitro-, Cyano-, Halogen-, Trifluormethyl-, Azido-, Formylamino-, oder Phenylsubstituiert sein kann,
eine $C_1$-$C_6$-Alkoxy-, $C_1$-$C_6$-Alkylamino-, Di-$C_1$-$C_6$-Alkylamino-, $C_1$-$C_6$-Alkylmercapto-, $C_1$-$C_6$-Alkylsulfinyl-, $C_1$-$C_6$-Alkylsufonyl-, $C_2$-$C_6$-Alkenyl-, $C_2$-$C_6$-Alkinyl-, $C_2$-$C_6$-Alkenyloxy-, $C_2$-$C_6$-Alkenylmercapto-, $C_2$-$C_6$-Alkinyloxy-, $C_2$-$C_6$-Alkinylmercapto-, $C_1$-$C_6$-Alkylcarbonylamino-, Di-$C_1$-$C_6$-Alkylcarbonylamino-, $C_1$-$C_6$-Alkylaminocarbonyl-, Formyl-, $C_1$-$C_6$-Alkylcarbonyl-, Carboxyl-, $C_1$-$C_6$-Alkoxycarbonyl-, $C_1$-$C_6$-Alkenyloxycarbonyl-, $C_1$-$C_6$-Alkinyloxycarbonyl-, Carboxy- $C_1$-$C_6$-alkyl-, $C_1$-$C_6$-Alkyloxycarbonyl-$C_1$-$C_6$-alkyl-, $C_2$-$C_6$-Alkenyloxycarbonyl- $C_1$-$C_6$-alkyl-, $C_2$-$C_6$-Alkinyloxycarbonyl- $C_1$-$C_6$-alkylgruppe,
Phenyl, das gegebenenfalls ein oder mehrfach unabhängig voneinander durch Hydroxy-, Amino-, Mercapto-, $C_1$-$C_6$-Alkoxy-, $C_1$-$C_6$-Alkylamino-, Di-$C_1$-$C_6$-Alkylamino-, $C_1$-$C_6$-Alkylmercapto-, $C_1$-$C_6$-Alkylsulfinyl-, $C_1$-$C_6$-Alkylsufonyl-, $C_2$-$C_6$-Alkenyl-, $C_2$-$C_6$-Alkinyl-, $C_2$-$C_6$-Alkenyloxy-, $C_2$-$C_6$-Alkenylmercapto-, $C_2$-$C_6$-Alkinyloxy-, $C_2$-$C_6$-Alkinylmercapto-, $C_1$-$C_6$-Alkylcarbonylamino-, $C_1$-$C_6$-Alkylaminocarbonyl-, Formyl-, $C_1$-$C_6$-Alkylcarbonyl-, Carboxyl-, $C_1$-$C_6$-Alkoxycarbonyl-, $C_1$-$C_6$-Alkenyloxycarbonyl-, $C_1$-$C_6$-Alkinyloxycarbonyl-, Carboxy- $C_1$-$C_6$-alkyl-, $C_1$-$C_6$-Alkyloxycarbonyl-$C_1$-$C_6$-alkyl-, $C_2$-$C_6$-Alkenyloxycarbonyl- $C_1$-$C_6$-alkyl-, $C_2$-$C_6$-Alkinyloxycarbonyl- $C_1$-$C_6$-alkyl-, Benzyloxy-, Phenylmercapto-Phenyloxy-, Nitro-, Cyano-, Halogen-, Trifluormethyl-, Azido-, Formylamino-, Carboxy- oder Phenyl- substituiert sein kann,
einen carbocyclischen mono-, bi- oder tricyclischen Rest mit 7-15 C-Atomen oder ein heterocyclisches mono-, bi- oder tricyclisches Ringsystem darstellen kann, wobei die ungesättigten bzw. aromatischen Carbo- und Heterocyclen partiell oder vollständig hydriert sein können.

$R_7$ symbolisiert einen
$C_1$-$C_6$-Alkyl-, Carboxyl-, Carboxoureido-, $C_1$-$C_6$-Alkoxycarbonyl-, $C_2$-$C_6$-Alkenyloxycarbonyl-, $C_2$-$C_6$-Alkinyloxycarbonyl-, Aminocarbonyl-, $C_1$-$C_6$-Alkylaminocarbonyl-, $C_2$-$C_6$-Alkenyloxycarbonyl- $C_1$-$C_6$-alkyl-, $C_2$-$C_6$-Alkinyloxycarbonyl- $C_1$-$C_6$-alkyl-, *N*-Hydroxyaminocarbonyl-, Hydroxamin-, *N*-$C_1$-$C_6$-Alkoxyaminocarbonyl-, *N*-$C_1$-$C_6$-Alkyl-*N*-Hydroxyaminocarbonyl-, *N*-$C_1$-$C_6$-Alkyl-*N*-$C_1$-$C_6$-Alkoxyaminocarbonyl-, Mercapto-, $C_1$-$C_6$-Alkylmercapto-, Phenylmercapto-, Pyridylmercapto, Halogen-, Dihydroxyboryl-, Di-$C_1$-$C_6$-Alkoxyboryl-, Sulfonsäure-, Phosphinsäure-, Phosphonsäure-, oder Barbitursäurerest.

$R_8$ stellt

Wasserstoff, oder einen $C_1$-$C_6$-Alkyl-, $C_2$-$C_6$-Alkenyl-, $C_2$-$C_6$-Alkinyl-, $C_1$-$C_6$-Alkoxycarbonyl-, Phenyl-, Pyridyl-, Thiophenylrest dar.

$R_9$ bedeutet Wasserstoff, Benzyl, Phenyl, oder $C_1$-$C_6$ Alkyl.

$R_{10}$ bedeutet
Wasserstoff, Carboxyl-, $C_1$-$C_6$-Alkoxycarbonyl-, $C_2$-$C_6$-Alkenyloxycarbonyl-, $C_2$-$C_6$-Alkinyloxycarbonyl-, Aminocarbonyl-, $C_1$-$C_6$-Alkylaminocarbonyl-, $C_2$-$C_6$-Alkenyloxycarbonyl- $C_1$-$C_6$-alkyl-, $C_2$-$C_6$-Alkinyloxycarbonyl- $C_1$-$C_6$-alkyl-, *N*-Hydroxyaminocarbonyl-, *N*-$C_1$-$C_6$-Alkoxyaminocarbonyl-, *N*-$C_1$-$C_6$-Alkyl-*N*-Hydroxyaminocarbonyl-, *N*-$C_1$-$C_6$-Alkyl-*N*-$C_1$-$C_6$-Alkoxyaminocarbonyl-,

Ein aliphatischer Rest bedeutet einen geradkettigen, verzweigten oder cyclischen Alkyl-, Alkenyl- oder Alkinylrest mit 1-15, vorzugsweise 1-10 Kohlenstoffatomen, wie z. B. der Methyl-, Ethyl-, Propyl-, Isopropyl-, Butyl-, *sec*-Butyl-, *tert*-Butyl-, Pentyl-, Hexyl-, Heptyl-, Oktyl-, Nonyl-, Decyl-, Cyclopropyl-, Cyclobutyl-, Cyclohexylrest. Ungesättigte Reste sind z. B. der Vinyl-, Allyl-, Dimethylallyl-, Butenyl-, Isobutenyl-, Pentenyl-, Ethinyl- oder Propinylrest.

$C_1$-$C_6$-Alkylreste in $C_1$-$C_6$-Alkyl, $C_1$-$C_6$-Alkoxy-, $C_1$-$C_6$-Alkylamino-, Di-$C_1$-$C_6$-Alkylamino, $C_1$-$C_6$-Alkylmercapto-, $C_1$-$C_6$-Alkylsulfinyl-, $C_1$-$C_6$-Alkylsufonyl-, $C_1$-$C_6$-Alkylcarbonylamino-, $C_1$-$C_6$-Alkylaminocarbonyl-, $C_1$-$C_6$-Alkoxycarbonyl-, $C_1$-$C_6$-Alkoxycarbonyl, Carboxy- $C_1$-$C_6$-alkyl-, $C_1$-$C_6$-Alkyloxycarbonyl-$C_1$-$C_6$--alkyl-, $C_2$-$C_6$-Alkenyloxycarbonyl-$C_1$-$C_6$-alkyl-, $C_2$-$C_6$-Alkinyloxycarbonyl- $C_1$-$C_6$-alkyl-, bedeuten geradkettige, verzweigte oder cyclische Reste. Bevorzugt sind die Methyl-, Ethyl-, Propyl-, Isopropyl-, Butyl-, *sec*- oder *tert*-Butyl-, Pentyl-, Hexyl-, Cyclopropyl-, Cyclobutyl-, Cyclohexyl-, Methoxy-, Ethoxy-, Propyloxy-, Isopropyloxy-, Butyloxy-, *sec*-Butyl-, *tert*-Butyloxy-, Methoxycarbonyl-, Ethoxycarbonyl-, Propyloxycarbonyl-, Butyloxycarbonyl-, Carboxymethyl-, Carboxyethyl-, Carboxypropyl-, Methoxycarbonylethyl-, Ethoxycarbonylethyl-, Methoxycarbonylpropyl-, Ethoxycarbonylpropyl-, Carboxymethoxy-, Carboxyethoxy-, Carboxypropyloxy-, Methoxycarbonylmethoxy-, Ethoxycarbonylethoxy-, Propoxycarbonylmethoxy-, Methoxycarbonylethoxy-, Ethoxycarbonylethoxy-, Aminomethyl-, Aminoethyl-, Aminopropyl-, Methylmercapto-, Ethylmercapto-, Propylmercaptogruppe.

$C_2$-$C_6$- Alkenyl- und $C_2$-$C_6$- Alkinylreste in $C_2$-$C_6$-Alkenyl, $C_2$-$C_6$-Alkinyl, $C_2$-$C_6$-Alkenyloxy, $C_2$-$C_6$-Alkenylmercapto, $C_2$-$C_6$-Alkinyloxy, $C_2$-$C_6$-Alkinylmercapto-, $C_2$-$C_6$-Alkenyloxycarbonyl-, $C_2$-$C_6$-Alkinyloxycarbonyl, $C_2$-$C_6$-Alkenyloxycarbonyl- $C_1$-$C_6$-alkyl-, $C_2$-$C_6$-Alkinyloxycarbonyl- $C_1$-$C_6$-alkyl-, bedeuten geradkettige, verzweigte oder cyclische Reste. Bevorzugt sind die Vinyl-, Propenyl-, Butenyl-, Pentenyl-, Hexenyl-, Ethinyl-, Propargyl-, Vinyloxy-, Allyloxy-, Propargyloxy-, Allyloxycarbonyl-, Propargyloxycarbonyl-, Allyloxycarbonylmethyl-, Allyloxycarbonylethyl-, Allyloxycarbonylpropyl-, Propargyloxycarbonylmethyl-, Propargyloxycarbonylethyl- und die Propargyloxycarbonylpropylgruppe.

Ein carbocyclischer Ring mit 7-15 C-Atomen kann mono-, bi- oder tricyclisch sein und pro Ring jeweils 5-7 C-Atome aufweisen. Dieser Ring kann aromatisch oder ganz oder teilweise gesättigt sein. Bevorzugt sind der Naphtyl-, Anthracenyl-, Phenanthrenyl-, Fluorenyl-, Indenyl-, Acenaphtylenyl-, Norbornyl-, Adamantylring oder eine $C_3$-$C_7$-Cycloalkyl oder $C_5$-$C_8$-Cycloalkenylgruppe. Der carbocyclische Ring kann darüberhinaus 1-3 fach substituiert sein, wobei die Substituenten unabhängig voneinander ein gerader oder verzweigter, gesättigter oder ungesättigter aliphatischer Rest mit 1-9 Kohlenstoffatomen, eine Hydroxy-, Amino-, Thio-, $C_1$-$C_6$-Alkoxy-, $C_1$-$C_6$-Alkylamino-, Di-$C_1$-$C_6$-Alkylamino-, $C_1$-$C_6$-Alkylmercapto-, $C_1$-$C_6$-Alkylsulfinyl-, $C_1$-$C_6$-Alkylsufonyl-, $C_2$-$C_6$-Alkenyl-, $C_2$-$C_6$-Alkinyl-, $C_2$-$C_6$-Alkenyloxy-, $C_2$-$C_6$-Alkenylmercapto-, $C_2$-$C_6$-Alkinyloxy-, $C_2$-$C_6$-Alkinylmercapto-, $C_1$-$C_6$-Alkylcarbonylamino-, $C_1$-$C_6$-Alkylaminocarbonyl-, Carbonyl-, $C_1$-$C_6$-Alkylcarbonyl-, Carboxyl-, $C_1$-$C_6$-Alkoxycarbonyl-, $C_1$-$C_6$-Alkenyloxycarbonyl-, $C_1$-$C_6$-Alkinyloxycarbonyl-, Carboxy- $C_1$-$C_6$-alkyl-, $C_1$-$C_6$-Alkyloxycarbonyl-$C_1$-$C_6$-alkyl-, $C_2$-$C_6$-Alkenyloxycarbonyl- $C_1$-$C_6$-alkyl-, $C_2$-$C_6$-Alkinyloxycarbonyl- $C_1$-$C_6$-alkyl-, Benzyloxy-, Phenylmercapto-, Phenyloxy-, Nitro-, Cyano-, Halogen-, Trifluormethyl-, Azido-, Formylamino-, oder Phenyl- sein können.

Unter einem heterocyclischen mono-, bi- oder tricyclischen Ringsystem versteht man ein gesättigtes oder ungesättigtes Ringsystem mit 5 bis 7 Ringgliedern, welches 1-3 gleiche oder verschiedene Heteroatome wie Stickstoff Sauerstoff oder Schwefel enthält, wie z.B. das Pyridin-, Pyrimidin-, Pyridazin-, Pyrazin-, Triazin-, Pyrrol-, Pyrazol-, Imidazol-, Triazol-, Thiazol-, Oxazol-, Isoxazol-, Oxadiazol-, Furazan-, Furan-, Thiophen-, Indol-, Chinolin- Isochinolin-, Cumaron-, Thionaphten-, Benzoxazol-, Benzthiazol-, Indazol-, Benzimidazol-, Benztriatol-, Chromen-, Phtalazin- Chinazolin-, Chinoxalin-, Methylendioxybenzol- Carbazol-, Acridin-, Phenoxazin-, Phenothiazin-, Phenazin-, oder Purinsystem, wobei die ungesättigten bzw. aromatischen Carbo- und Hetercyclen partiell oder vollständig hydriert sein können. Das heterocyclische System kann darüberhinaus ein oder mehrfach substituiert sein, wobei die Substituenten unabhängig voneinander ein gerader oder verzweigter, gesättigter oder ungesättigter aliphatischer Rest mit 1-9 Kohlenstoffatomen, eine Hydroxy-, Amino-, Thio-, $C_1$-$C_6$-Alkoxy-, $C_1$-$C_6$-Alkylamino-, Di-$C_1$-$C_6$-Alkylamino-, $C_1$-$C_6$-Alkylmercapto-, $C_1$-$C_6$-Alkylsulfinyl-, $C_1$-$C_6$-Alkylsufonyl, $C_2$-$C_6$-Alkenyl-, $C_2$-$C_6$-Alkinyl-, $C_2$-$C_6$-Alkenyloxy-, $C_2$-$C_6$-Alkenylmercapto-, $C_2$-$C_6$-Alkinyloxy-, $C_2$-$C_6$-Alkinylmercapto-, $C_1$-$C_6$-Alkylcarbonylamino-, $C_1$-$C_6$-Alkylaminocarbonyl-, Carbonyl-, $C_1$-$C_6$-Alkylcarbonyl-, Carboxyl-, $C_1$-$C_6$-Alkoxycarbonyl-, $C_1$-$C_6$-Alkenyloxycarbonyl-, $C_1$-$C_6$-Alkinyloxycarbonyl-, Carboxy- $C_1$-$C_6$-alkyl-, $C_1$-$C_6$-Alkyloxycarbonyl-$C_1$-$C_6$-alkyl-, $C_2$-$C_6$-Alkenyloxycarbonyl- $C_1$-$C_6$-alkyl-, $C_2$-$C_6$-Alkinyloxycarbonyl- $C_1$-

$C_6$-alkyl-, Benzyloxy-, Phenylmercapto-, Phenyloxy-, Nitro-, Cyano-, Halogen-, Trifluormethyl-, Azido-, Formylamino-, oder Phenyl- sein können. Bevorzugt sind Pyrrolidin, Piperidin, Piperazin, Morpholin, Hexahydroazepin, Tetrahydrofuran, Tetrahydropyran, Tetrahydrothiophen, 4,5-Dihydroimidazol, Pyrrol, Imidazol, Pyrazin, Pyrimidin, Pyridazin, 1*H*-Azepin, 3*H*-Azepin, 1,2-Diazepin, 1,4-Diazepin, Furan, Thiophen, Oxazol, Isoxazol, Thiazol, Isothiazol, Pyrazol, Pyrollidinon, Imidazolidinon, Piperidinon, Indol, Purin, Chinolin und Isochinolin.

Insbesondere bevorzugt sind Verbindungen der allgemeinen Formel I bzw. II, in denen $R_1$ und/oder $R_3$ einen $C_1$-$C_6$-Alkyl-, einen $C_1$-$C_6$-Alkoxycarbonyl-, einen Carboxyl- oder einen formylsubstituierten $C_2$-$C_4$-Alkenylrest bedeutet, $R_2$ Wasserstoff, Amino, $C_1$-$C_6$-Alkylcarbonylamino, Di-$C_1$-$C_6$-Alkylcarbonylamino, $C_1$-$C_6$-Alkoxy oder Halogen bedeutet, $R_4$ gleich Wasserstoff Halogen, $(CH_2)_nCH_2R_7$ mit n gleich 0, $R_7$ gleich $C_1$-$C_6$-Alkoxycarbonyl, $R_7$ gleich Carboxyl, $R_7$ gleich N-Hydroxyaminocarbonyl, oder $R_4$ gleich $(CH_2)_nCHR_7R_8$ mit n gleich 0, $R_7$ und $R_8$ gleich $C_1$-$C_6$-Alkoxycarbonyl-, oder $R_7$ gleich Carboxoureido und $R_8$ gleich $C_1$-$C_6$-Alkoxycarbonyl oder mit n gleich 1, $R_7$ gleich Hydroxamin und $R_8$ gleich Alkoxycarbonyl, oder $R_4$ gleich Mercapto bedeutet und $R_5$ und/oder $R_6$ bevorzugt Wasserstoff oder $C_1$-$C_6$-Alkyl bedeuten.

Ferner sind besonders bevorzugt Verbindungen, in denen $R_1$ und $R_3$ Ethoxycarbonyl- und $R_5$ und $R_6$ Wasserstoff bedeuten.

Unter den physiologisch verträglichen Salzen der Azulenderivate sowie der Verbindungen der allgemeinen Formel I bzw. II versteht man beispielsweise Formiate, Acetate, Caproate, Oleate, Lactate oder Salze von Carbonsäuren mit bis zu 18 Kohlenstoffatomen oder Salze von Dicarbonsäuren und Tricarbonsäuren wie Citrate, Malonate und Tartrate oder Alkansulfonate mit bis zu 10 Kohlenstoffatomen oder p-Toluolsulfonate oder Salicylate oder Trifluoracetate oder Salze von physiologisch verträglichen Mineralsäuren wie Salzsäure, Bromwasserstoffsäure, Iodwasserstoffsäure, Schwefelsäure, Phosphorsäure. Die Azulenderivate sowie die Verbindungen der Formel I bzw. II mit freier Carboxylgruppe können auch Salze mit physiologisch verträglichen Basen bilden. Beispiele solcher Salze sind Alkalimetall-, Erdalkalimetall-, Ammonium- und Alkylammoniumsalze, wie das Natrium, Kalium-, Calcium- oder Tetramethylammoniumsalz. Zu reinen Enantiomern der Azulenderivate sowie der Verbindungen der Formel I bzw. II kommt man entweder durch Racematspaltung (über Salzbildung mit optisch aktiven Säuren oder Basen), oder indem man in die Synthese optisch aktive Ausgangsstoffe einsetzt.

Die Verbindungen der allgemeinen Formel I bzw. II können solvatisiert, insbesondere hydratisiert sein. Die Hydratisierung kann im Zuge der Herstellung erfolgen oder allmählich als Folge hygroskopischer Eigenschaften einer zunächst wasserfreien Verbindung auftreten.

Die erfindungsgemäßen Verbindungen können in flüssiger, fester oder in Form von Aerosolen oral, enteral, parenteral, topisch, nasal, pulmonal oder rectal in allen üblichen nichttoxischen pharmazeutisch akzeptierten Trägermaterialien, Adjuvantien und Zusätzen verabbreicht werden. Der Begriff parenteral umfaßt dabei subcutane, intravenöse und intramuskuläre Zufuhr oder Infusionen. Orale Applikationsformen sind z.B. in W.A. Ritschel, Die Tablette, 1966 Aulendorf, beschrieben und können z.B. Tabletten, Kapseln, Dragees, Sirupe, Lösungen, Suspensionen, Emulsionen, Elixiere etc. sein, die einen oder mehrere Zusätze aus den folgenden Gruppen enthalten können, wie z.B. Geschmacksstoffe, Süßstoffe, Farbstoffe und Konservierungsmittel. Orale Applikationsformen enthalten den wirksamen Bestandteil zusammen mit nichttoxischen, pharmazeutisch akzeptierten Trägermaterialien, die zur Herstellung von Tabletten, Kapseln, Dragees usw. geeignet sind, wie z.B. Calciumcarbonat, Natriumcarbonat, Lactose, Calciumphosphat oder Natriumphosphat; Stärke, Mannit, Methylcellulose, Talkum, hochdisperse Kieselsäuren, höhermolekulare Fettsäuren (wie Stearinsäure), Erdnußöl, Olivenöl, Paraffin, Miglyol, Gelatine, Agar-Agar, Magnesiumstearat, Bienenwachs, Cetylalkohol Lecithin, Glycerol, tierische und pflanzliche Fette, feste hochmolekulare Polymere (wie Polyethylenglykole). Tabletten, Kapseln, Dragees usw. können mit einem entsprechenden Überzug, wie z.B. Glycerylmonostearat oder Glyceryldistearat versehen werden, so daß unerwünschte Nebenwirkungen im Magen verhindert werden, oder es durch die verzögerte Absorption im Gastrointestinaltrakt zu einer längeren Wirkungsdauer kommt. Als Injektionsmedium kommen vorzugsweise sterile injizierbare wäßrige oder ölige Lösungen oder Suspensionen zur Anwendung, welche die üblichen Zusätze, wie Stabilisierungsmittel und Lösungsvermittler enthalten. Deratige Zusätze können z.B. Wasser, isotonische Kochsalzlösung, 1,3-Butandiol, Fettsäuren (wie Ölsäure), Mono- und Diglyceride oder Miglyol sein. Für die rectale Anwendung können alle geeigneten nicht irritierenden Zusätze verwendet werden, die bei normalen Temperaturen fest und bei Rectaltemperatur flüssig sind wie z. B. Kakaobutter und Polyethylenglykol. Für die Anwendung als Aerosol kommen die pharmazeutisch üblichen Trägermedien zur Anwendung. Für den äußerlichen Gebrauch finden Cremes, Tinkturen, Gele, Lösungen oder Suspensionen usw. mit den pharmazeutisch üblichen Zusätzen Anwendung.

Die Dosierung kann von verschiedenen Faktoren, wie Applikationsweise, Spezies, Alter und/oder individuellem Zustand abhängen. Die täglich zu verabreichende Dosis wirksamer Substanz liegt bei 0.01 mg bis ungefähr 100 mg/kg Körpergewicht, vorzugsweise bei 0.1 bis 10 mg/kg Körpergewicht und kann auf einmal oder mehrere Male verteilt appliziert werden.

Verbindungen der allgemeinen Formel I bzw. II in denen die Reste $R_1$ bis $R_6$ die angegebene Bedeutung haben können nach an sich literaturbekannten Verfahren dargestellt werden (siehe K.-P. Zeller „Azulene" in Methoden der

organischen Chemie (Houben - Weyl), Band V/2c, Seite 127).
Z. B. werden solche Verbindungen aus entsprechend substituierten Troponen (Siehe zur Synthese: T. Asao, M. Oda, „Tropone, Tropolone und deren Derivate" in Methoden der organischen Chemie (Houben -Weyl), Band V/2c Seite 710) erhalten. (siehe z. B.: T. Nozoe, H. Takeshita, K. Tajiri; *Bull. Chem. Soc. Jpn*. **56**, 3679 und darin zitierte Literatur)

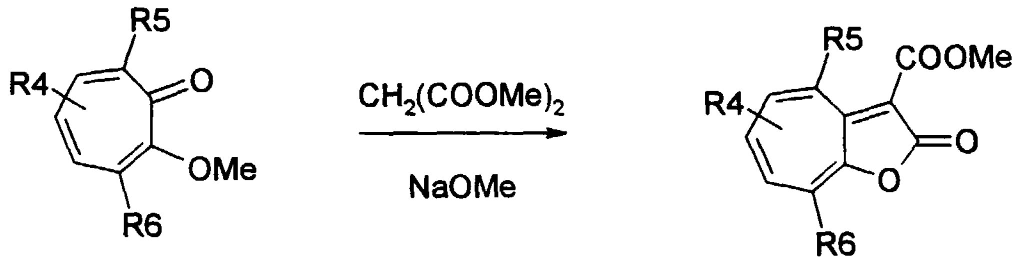


Die so erhaltenen 2H-Cyclohepta [b] furan-2-one lassen sich mit aktiven Methylencomponenten (z. B. Essigsäurederivaten (T. Nozoe, K. Takase, T. Nakazawa, S. Sugita, *Bull. Chem. Soc Jap.* **1974**, 1750 und dort zitierte Literatur) oder Enaminen (z. B. M. Yasunami, A. Chen, P. W. Wang, K. Takase, *Chem. Lett*. **1980**, 579)) zu den entsprechenden Azulenen umsetzen.

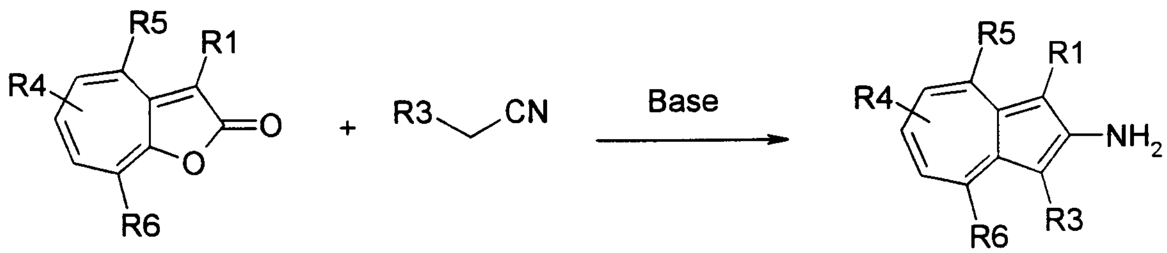


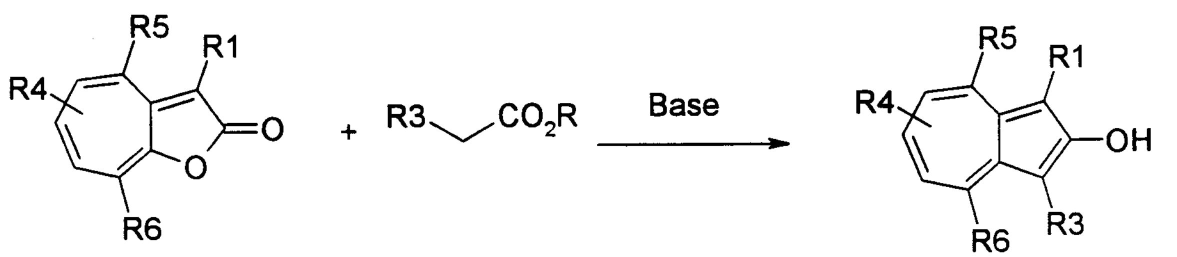


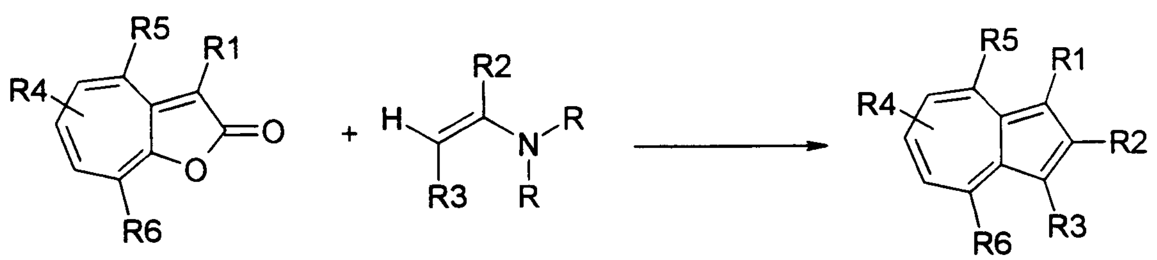

Die so erhaltenen Aminoazulene können nach an sich bekannten Verfahren zu verschiedenen Azulenderivaten in denen die Reste $R_1$-$R_6$ die angegebene Bedeutung haben umgesetzt werden. Beispielsweise kann nach Nitrosierung die Aminogruppe durch Halogen ersetzt werden (z. B.: T. Nozoe, S. Seto, S. Matsumura, *Bull. Chem. Soc. Jap.* **1962**,

1990). Auch die gezeigten Hydroxyderivate in denen Die Reste $R_1$ und $R_3$-$R_6$ die angegebene Bedeutung haben, sind vielseitige Ausgangsstoffe. Sie reagieren nach Methylierung z. B. mit Grignard-Verbindungen zu den entsprechenden alkylierten Produkten (D. Balschukat, E. V. Dehmlow, *Chem. Ber.* **1986**, 2272).

Weiterhin bevorzugt außer den in den Beispielen aufgeführten Verbindungen ist die Verwendung der folgenden literaturbekannten Azulenderivate als Metalloproteaseinhibitoren:

**Beispiel 1:**

2-Amino-6-ethoxycarbonylmethyl-azulen-1,3-dicarbonsäurediethylester

Die Verbindung ist erhältlich nach: Dehmlow, Eckehard V.; Balschukat, Dietmar: Schmidt, Peter P.; Groening, Carsten; *Chem.Ber.*; **1986**; 2956.

**Beispiel 2:**

2-Diacetylamino-6-ethoxycarbonylmethyl-azulen-1,3-dicarbonsäurediethylester

Die Verbindung ist durch Acetylierung erhältlich aus 2-Amino-6-ethoxycarbonylmethylazulen-1,3-dicarbonsäurediethylester. (E. V. Dehmlow, private Mitteilung)

**Beispiel 3:**

6-(Bis-ethoxycarbonylmethyl)-2-diacetylamino -azulen-1,3-dicarbonsäurediethylester

Die Verbindung entsteht als Zwischenprodukt bei der Synthese von 2-Amino-6-ethoxycarbonylmethyl-azulen-1,3-dicarbonsäurediethylester (Dehmlow, Eckehard V.; Balschukat, Dietmar; Schmidt, Peter P.; Groening, Carsten; *Chem.Ber.*; **1986**; 2956).

**Beispiel 4:**

6-Bromo-2-diacetylamino-azulen-1,3-dicarbonsäurediethylester

Die Verbindung ist erhältlich nach: Tada,M.; *Bull.Chem.Soc.Jpn.*; **1966**; 1954.

**Beispiel 5:**

2-Acetylamino-6-bromo-azulen-1,3-dicarbonsäurediethylester

Die Verbindung ist erhältlich nach: Tada,M.; *Bull.Chem.Soc.Jpn.*; **1966**; 1954.

**Beispiel 6:**

2-Ethoxy-6-ethoxycarbonylmethyl-azulen-1,3-dicarbonsäurediethylester

Die Verbindung ist erhältlich nach: Balschukat, Dietmar; Dehmlow, Eckehard V.; *Chem.Ber.*; **1986**; 2272.

**Beispiel 7:**

2-Amino -azulen-1,3-dicarbonsäurediethylester

Die Verbindung ist erhältlich nach: Nozoe et al.: *Proc.Jpn.Acad.*; **1956**; 339.

**Beispiel 8:**

2-Amino-6-mercapto-azulen-1,3-dicarbonsäurediethylester

Die Verbindung ist erhältlich nach: Tada,M.; *Bull. Chem.Soc.Jpn.*; **1966**; 1954.

**Beispiel 9:**

2-Chloro-6-(2-ethoxycarbonyl-2-hydroxyimino-ethyl)-azulen-1,3-dicarbonsäurediethylester

Die Verbindung ist erhältlich nach: Dehmlow, Eckehard V.; Balschukat, Dietmar; Schmidt, Peter P.; Groening, Carsten; *Chem.Ber.*; **1986**; 2956.

**Beispiel 10:**

3-(5-isopropyl-3,8-dimethyl-azulen-1-yl)-propenal

Die Verbindung ist erhältlich nach: Asato, Alfred E.; Peng, Ao; Hossain, Mohammad Z.; Mirzadegan, Taraneh; Bertram, John S.; *J.Med.Chem.*; **1993**; 3137.

**Beispiel 11:**

Periphere humane Leukozyten werden mit LPS über 22 Std. inkubiert. Der Kultutüberstand wird mit einem TNF$\alpha$ spezifischen Enzym-Immuno-Assay untersucht.
Durch Vergleich der für die Messproben erhaltenen TNF$\alpha$-Konzentrationen mit den ungehemmten Proben wird die prozentuale Hemmung für die einzelne Probe berechnet. Aus der Konzentrationsabhängigkeit der Hemmwerte werden rechnerisch die $IC_{50}$-Werte bestimmt.

Der $IC_{50}$-Wert läßt sich wie folgt ermitteln:

$$v = v_0/(1 + [I] / IC_{50})$$

v = Anfangsgechwindigkeit
$V_0$ = Anfangsgeschwindigkeit ohne Inhibitor
[I] = Inhibitorkonzentration

Die $IC_{50}$-Werte der oben genannten Substanzen, die Gegenstand dieses Patentes sind, gegen TNF-$\alpha$ finden sich in der folgenden Tabelle;

| Verbindung | $IC_{50}$ [$\mu$M] |
|---|---|
| 1 | 0.2 |
| 2 | 0.05 |
| 3 | 0.18 |
| 4 | 1.3 |
| 5 | 1.1 |
| 6 | 11.9 |
| 7 | 25.4 |
| 8 | 26.0 |
| 9 | 2.7 |
| 10 | 10.7 |

**Patentansprüche**

1. Verwendung von Azulenderivaten sowie deren Tautomere, Enantiomere, Diastereomere, Racemate und physiologisch verträglichen Salze oder Ester als Metalloproteaseinhibitoren.

2. Verwendung von Azulenderivaten der allgemeinen Formel I bzw. II als Metalloproteaseinhibitoren

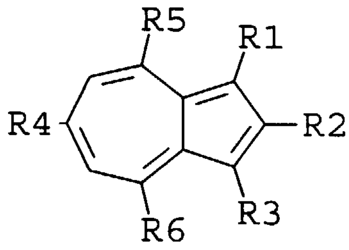

I

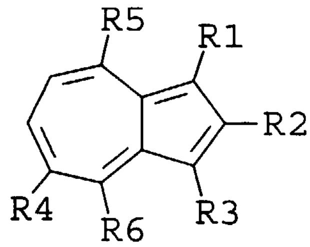

II

wobei

$R_1$, $R_2$ und $R_3$ einzeln oder unabhängig voneinander
Wasserstoff Hydroxy-, Halogen-, Amino-, Mercapto-, ein gerader oder verzweigter, gesättigter oder ungesättigter aliphatischer Rest mit 1 - 15 Kohlenstoffatomen, der ein oder mehrfach substituiert sein kann, eine Alkoxy-, Alkylamino-, Di- Alkylamino-, Alkylmercapto-, Alkylsulfinyl-, Alkylsufonyl-, Alkenyl-, Alkinyl-, Alkenyloxy-, Alkenylmercapto-, Alkinyloxy-, Alkinylmercapto-, Alkylcarbonylamino-, Di-N-Alkylcarbonylamino-, Alkylaminocarbonyl-, Formyl-, Alkylcarbonyl-, Carboxyl-, Alkoxycarbonyl-, Alkenyloxycarbonyl-, Alkinyloxycarbonyl-, Carboxyalkyl-, Alkyloxycarbonylalkyl, Alkenyloxycarbonylalkyl-, Alkinyloxycarbonylalkyl-, Nitro-, Cyano-, Halogen-, Trifluormethyl-, Azidogruppe, Phenyl, oder Phenylcarbonyl, das gegebenenfalls ein oder mehrfach unabhängig voneinander substituiert sein kann, einen carbocyclischen mono-, bi- oder tricyclischen Rest mit 7-15 C-Atomen oder ein heterocyclisches mono-, bi- oder tricyclisches Ringsystem bedeuten.

$R_4$ im Fall von Verbindungen der allgemeinen Formel I
Wasserstoff Halogen, $(CH_2)_nCH_2R_7$, $(CH_2)_nCHR_7R_8$, $CH(OR_8)R_7$, $CH(SR_8)R_7$, $NR_9R_{10}$, $OR_{10}$, $SR_{10}$, $C(O)R_{10}$,
im Fall von Verbindungen der allgemeinen Formel II $R_7$, $(CH_2)_nCH_2R_7$, $(CH_2)_nCHR_7R_8$, $CH(OR_8)R_7$, $CH(SR_8)R_7$, $NR_9R_{10}$, $OR_{10}$, $SR_{10}$, $C(O)R_{10}$,

n die Zahl 0 oder 1 bedeutet

$R_5$ und $R_6$ einzeln und unabhängig voneinander
Wasserstoff ein gerader oder verzweigter, gesättigter oder ungesättigter aliphatischer Rest mit 1-15 Kohlenstoffatomen, der ein oder mehrfach substituiert sein kann, eine Alkoxy-, Alkylamino-, Di- Alkylamino-, Alkylmercapto-, Alkylsulfinyl-, Alkylsufonyl-, Alkenyl-, Alkinyl-, Alkenyloxy-, Alkenylmercapto-, Alkinyloxy-, Alkinylmercapto-, Alkylcarbonylamino-, Di- Alkylcarbonylamino-, Alkylaminocarbonyl-, Formyl-, Alkylcarbonyl-, Carboxyl-, Alkoxycarbonyl-, Alkenyloxycarbonyl-, Alkinyloxycarbonyl-, Carboxyalkyl-, Alkyloxycarbonylalkyl, Alkenyloxycarbonylalkyl-, Alkinyloxycarbonylalkyl-, Nitro-, Cyano-, Halogen-, Trifluormethyl-, Azidogruppe, Phenyl, oder Phenylcarbonyl, das gegebenenfalls ein oder mehrfach unabhängig voneinander substituiert sein kann, einen carbocyclischen mono-, bi- oder tricyclischen Rest mit 7-15 C-Atomen oder ein heterocyclisches mono-, bi- oder tricyclisches Ringsystem,

$R_7$,
Alkyl-, Carboxyl-, Carboxoureido-, Alkoxycarbonyl-, Alkenyloxycarbonyl-, Alkinyloxycarbonyl-, Aminocarbonyl-, Alkylaminocarbonyl-, Alkenyloxycarbonylalkyl-, Alkinyloxycarbonylalkyl-, *N*-Hydroxyaminocarbonyl-, *N*-Alkoxyaminocarbonyl-, *N*-Alkyl-*N*-Hydroxyaminocarbonyl-, Hydroxamin-, *N*-Alkyl-*N*-Alkoxyaminocarbonyl-, Mercapto-, Alkylmercapto-, Phenylmercapto-, Pyridylmercapto, Halogen-, Dihydroxyboryl-, Di-Alkoxyboryl-, Sulfonsäure-, Phosphonsäure-, Barbitursäure-,

$R_8$
Wasserstoff, Alkyl-, Alkenyl-, Alkinyl-, Phenyl-, Pyridyl-, Thiophenyl-, Alkoxycarbonyl-, und

$R_9$
Wasserstoff Benzyl, Phenyl, Alkyl bedeutet,
und

$R_{10}$
Wasserstoff Carboxyl-, Alkoxycarbonyl-, Alkenyloxycarbonyl-, Alkinyloxycarbonyl-, Aminocarbonyl-, Alkylaminocarbonyl-, Alkenyloxycarbonylalkyl-, Alkinyloxycarbonylalkyl, *N*-Hydroxyaminocarbonyl-, *N*-Alkoxyaminocarbonyl-, *N*-Alkyl-*N*-Hydroxyaminocarbonyl-, *N*-Alkyl-*N*-Alkoxyaminocarbonyl- bedeutet

sowie deren Tautomere, Enantiomere, Diastereomere, Racemate und physiologisch verträglichen Salze oder Ester und Substanzen, die *in vivo* zu Verbindungen der Formel I bzw. II hydrolysiert oder metabolisiert werden.

**3.** Verwendung von Azulenderivaten gemäß Anspruch 2, dadurch gekennzeichnet daß

$R_1$ und/oder $R_3$ einen $C_1$-$C_6$-Alkyl-, einen $C_1$-$C_6$-Alkoxycarbonyl-, einen Carboxyl- oder einen formylsubstituierten $C_2$-$C_4$-Alkenylrest bedeutet, $R_2$ Wasserstoff, Amino, $C_1$-$C_6$-Alkylcarbonylamino, Di-$C_1$-$C_6$-Alkylcarbonylamino, $C_1$-$C_6$-Alkoxy oder Halogen bedeutet, $R_4$ gleich Wasserstoff, Halogen, $(CH_2)_nCH_2R_7$ mit n gleich 0, $R_7$ gleich $C_1$-$C_6$-Alkoxycarbonyl, $R_7$ gleich Carboxyl, $R_7$ gleich N-Hydroxyaminocarbonyl, oder $R_4$ gleich $(CH_2)_nCHR_7R_8$ mit n gleich 0, $R_7$ und $R_8$ gleich $C_1$-$C_6$-Alkoxycarbonyl-, oder $R_7$ gleich Carboxoureido und $R_8$ gleich $C_1$-$C_6$-Alkoxycarbonyl oder mit n gleich 1, $R_7$ gleich Hydroxamin und $R_8$ gleich $C_1$-$C_6$-Alkoxycarbonyl, oder $R_4$ gleich Mercapto bedeutet und $R_5$ und/oder $R_6$ bevorzugt Wasserstoff oder $C_1$-$C_6$-Alkyl bedeuten.

**4.** Verwendung von Azulenderivaten gemäß Anspruch 2 und 3, ausgewählt aus der Gruppe

2-Amino-6-ethoxycarbonylmethyl-azulen-1,3-dicarbonsäurediethylester
2-Diacetylamino-6-ethoxycarbonylmethyl-azulen-1,3-dicarbonsäurediethylester
6-(Bis-ethoxycarbonylmethyl)-2-diacetylamino -azulen-1,3-dicarbonsäurediethylester
6-Bromo-2-diacetylamino-azulen-1,3-dicarbonsäurediethylester
2-Acetylamino-6-bromo-azulen-1,3-dicarbonsäurediethylester
2-Ethoxy-6-ethoxycarbonylmethyl-azulen-1,3-dicarbonsäurediethylester
2-Amino -azulen-1,3-dicarbonsäurediethylester
2-Amino-6-mercapto-azulen-1,3-dicarbonsäurediethylester
2-Chloro-6-(2-ethoxycarbonyl-2-hydroxyimino-ethyl)-azulen-1,3-dicarbonsäurediethylester
3-(5-isopropyl-3,8-dimethyl-azulen-1-yl)-propenal

sowie deren Tautomere, Enantiomere, Diastereomere, Racemate und physiologisch verträglichen Salze oder Ester.

Europäisches Patentamt

# EUROPÄISCHER RECHERCHENBERICHT

Nummer der Anmeldung
EP 97 11 0534

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| A | EP 0 147 915 A (KOTOBUKI SEIYAKU CO. LTD.)<br>* Ansprüche 1-9 *<br>--- | 1-4 | A61K31/015 |
| A | EP 0 266 956 A (AJINOMOTO CO. INC.)<br>* Ansprüche 1-4 *<br>* Seite 5, Zeile 17 - Zeile 20 *<br>--- | 1-4 | |
| A | PATENT ABSTRACTS OF JAPAN<br>vol. 10, no. 245 (C-368), 22.August 1986<br>& JP 61 076432 A (AJINOMOTO CO INC),<br>18.April 1986,<br>* Zusammenfassung *<br>--- | 1-4 | |
| A,D | EP 0 497 192 A (F. HOFFMANN-LA ROCHE AG)<br>* Ansprüche 1,26,27,30,31 *<br>* Seite 1, Zeile 44 - Zeile 58 *<br>--- | 1-4 | |
| A,D | EP 0 489 577 A (CELLTECH LTD)<br>* Ansprüche 1,7,14 *<br>* Seite 11, Zeile 10 - Zeile 28 *<br>--- | 1-4 | RECHERCHIERTE SACHGEBIETE (Int.Cl.6) |
| A,D | WO 92 09563 A (GLYCOMED INC.)<br>* Ansprüche 1-8 *<br>----- | 1-4 | C07C<br>A61K |

Der vorliegende Recherchenbericht wurde für alle Patentansprüche erstellt

| Recherchenort | Abschlußdatum der Recherche | Prüfer |
|---|---|---|
| BERLIN | 26.November 1997 | Siatou, E |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)